# EUROPÄISCHE PATENTSCHRIFT

(11) **EP 1 455 745 B1**
(45) Veröffentlichungstag und Bekanntmachung des Hinweises auf die Patenterteilung: **02.07.2008**
(21) Anmeldenummer: 02792947.0
(22) Anmeldetag: 10.12.2002
(51) Int. Cl.: A61Q 15/00, A61Q 17/02, A61Q 17/04

(54) **Verwendung von Dimerdiolen**
Use of dimerdiols
Utilisation de dimerdiols

(30) Priorität: 19.12.2001 DE 10162697
(43) Veröffentlichungstag der Anmeldung: 15.09.2004
(73) Patentinhaber: Cognis IP Management GmbH, 40589 Düsseldorf (DE)
(72) Erfinder: ZANDER, Lars, 41569 Rommerskirchen (DE); KAWA, Rolf, 40789 Monheim (DE)
(86) Internationale Anmeldenummer: PCT/EP2002/013985
(87) Internationale Veröffentlichungsnummer: WO 2003/051324

(56) Entgegenhaltungen:
- WO-A-01/58578
- WO-A-96/26710
- DE-A- 19 631 792
- FR-A- 2 795 309

## Beschreibung

### Gebiet der Erfindung

Gegenstand der vorliegenden Erfindung ist die Verwendung von Dimerdiolen in kosmetischen/pharmazeutischen Sonnenschutzmitteln, Zubereitungen der dekorativen Kosmetik, Insektenschutzmittel oder Deo/Antitranspirant-Formulierungen zur Verbesserung der Wasserfestigkeit dieser Mittel.

### Stand der Technik

Obwohl Emulsionen seit langer Zeit bekannt sind, bestehen auf dem Kosmetikmarkt intensive Bemühungen sowohl die Stabilität als auch die sensorischen Eigenschaften dieser dispersen Systeme stets zu verbessem. Zu den gegenwärtigen Trends gehört unter anderem die Suche nach Ölkörpem und Polymeren, die sich leicht in Emulsionen einarbeiten lassen, die Formulierungen besonders lagerstabiler Emulsionen erlauben und sensorisch ein leichteres Hautgefühl vermitteln.

Für spezielle Applikafionsbereiche, wie beispielsweise Sonnenschutzmittel, spielt weiterhin die Wasserfestigkeit der Mittel eine wesentliche Rolle, da die UV-Lichtschutzfilter möglichst lange auf der Haut verweilen sollten ohne beim Baden abgewaschen zu werden. Die Wasserfestigkeit einer Sonnenschutzformulierung wird üblicherweise durch den Zusatz von Polymeren, wie beispielsweise PVP/Hexadecene Copolymer (Antaron^{®} V-216), erzielt. Diese Polymere haben aber den Nachteil, dass die Wasserfestigkeit nur über einen kurzen Zeitraum sichergestellt wird, und ein Langzeitschutz, wie er z.B. von Wassersportlem (Surfern) oder für den Sonnenschutz für Kinder gefordert wird, nicht erreichbar ist. Darüber hinaus wird die Sensorik der Emulsion hinsichtlich Einziehvermögen, Verteilbarkeit und Klebrigkeit deutlich verschlechtert.

DE 196 31 792 (Henkel) beschreibt Zubereitungen welche Ölkörper, Emulgatoren und UV-Lichtschutzfilter enthalten. Auf S.5 der D1 werden technische Dimerdiole als eine geeignete Klasse von Emulgatoren genannt. In D1 findet sich kein Hinweis darauf, dass Dimerdiole geeignet wären die Wasserfestigkeit von Sonnenschutzmitteln oder anderen kosmetischen Zubereitungen zu verbessern. WO 01/58578 (Seppic) beschreibt Zubereitungen, welche Umsetzungsprodukte von Alkylpolyglucosiden und Dimerdiolen enthalten und welche besonders geeignet sind, um Versprühbahre Zusammensetzungen herzustellen.

WO 03/051960 (Cognis, Stand der Technik nach Art. 54(3) EPÜ) beschreibt Sonnenschutzemulsionen, die Polylalkylenether mit C5-C100 Alkylengruppen und einer mittleren Molmasse von 300 bis 100 000 enthalten. Die Verwendung von Dimerdiolen zur Verbesserung der Wasserfestigkeit von Sonnenschutzmitteln oder anderen kosmetischen Zubereitungen ist nicht beschrieben. WO 03/041676 (Cognis, Stand der Technik nach Art. 54(3) EPÜ) beschreibt kosmetische Zubereitungen beschrieben, die Polycarbonate mit einer mittleren Molmasse von 300 bis 100 000 enthalten. Diese Polycarbonate sind beispielsweise erhältlich durch Umsetzung von Dimerdiolen mit Dialkylcarbonaten. Die Verwendung von Dimerdiolen zur Verbesserung der Wasserfestigkeit von Sonnenschutzmitteln oder anderen kosmetischen Zubereitungen ist nicht beschrieben.

Die Aufgabe der vorliegenden Erfindung hat darin bestanden, Sonnenschutzmittel zur Verfügung zu stellen, die einerseits eine verbesserte Sensorik, insbesondere hinsichtlich Einziehvermögen, Verteilbarkeit und Klebrigkeit aufweisen, andererseits im Vergleich zum Stand der Technik eine verbesserte Wasserfestigkeit aufweisen, die also bei Einarbeitung von Lichtschutzfiltern einen verbesserten Langzeitschutz bieten.

### Beschreibung der Erfindung

Es wurde nun überraschenderweise gefunden, dass Sonnenschutzmittel auf Basis von Dimerdiolen eine verbesserte Sensorik hinsichtlich Verteilbarkeit, Einziehvermögen und Klebrigkeit und eine verbesserte Wasserfestigkeit aufweisen, d.h. dass sie einen signifikant höheren UV-Schutz bieten.

Gegenstand der Erfindung ist daher die Verwendung von Dimerdiolen in Sonnenschutzmitteln, zur Verbesserung der Wasserfestigkeit der Sonnenschutzmittel. Die erfindungsgemäßen Mittel können als W/O oder O/W-Emulsionen oder auch als wasserfreie Sonnenschutzöle formuliert werden.

Die Verbesserung der Wasserfestigkeit ist nicht nur für Sonnenschutzformulierungen von großem Interesse, sondern auch für viele Bereiche der dekorativen Kosmetik relevant, wie beispielsweise Mascaras, Lidschatten, wasserfestes Make-up, Eyeliner, Kajalstifte, etc., des weiteren für Insektenschutzmittel und DeolAntitranspirant-Formulierungen. Auch für diese Applikationszwecke sind die Dimerdiole zur Verbesserung der Wasserfestigkeit vorteilhaft einsetzbar.

Gegenstand der Erfindung ist daher die Verwendung von Dimerdiolen zur Verbesserung der Wasserfestigkeit von Sonnenschutzmitteln, Zubereitungen der dekorativen Kosmetik, Insektenschutzmittel oder Deo/Antitranspirant Formulierungen.

### Dimerdiole

Dimerdiole sind herstellungsbedingt Gemische; ihre Herstellung ist hinreichend aus dem Stand der Technik bekannt, beispielsweise gemäß DE 1 768 313 und US 2,347,562. Kosmetische Zubereitungen mit einem Gehalt an technischen Dimerdiol/Trimertriol-Gemischen sind Gegenstand der WO 96/26710. Als Dimerdiol-Komponenten eignen sich vorzugsweise Dimerdiole mit einer Gesamtkohlenstoffzahl von C₁₂-C₁₀₀. Besonders gut geeignet sind C₁₂-C₄₀-Dimerdiole, vorzugsweise C₁₂-C₂₄- und besonders bevorzugt C₁₆-C₂₂-Dimerdiole, wobei sich die Angabe der C-Kettenlänge hier auf eine Kette bezieht. Die Verwendung von Dimerdiolen mit einer OH-Zahl von 200 - 220 ist erfindungsgemäß bevorzugt. Eine weitere erfindungsgemäß bevorzugte Ausführungsform ist die Verwendung von hydrierte Dimerdiole mit Jodzahlen von 20 bis 80, vorzugsweise 50 bis 70. Erfindungsgemäß besonders bevorzugt sind Pripol^{®} 2033 (Uniqema) und Sovermol^{®} 908 (Cognis Deutschland GmbH).

Üblicherweise werden die Dimerdiole in Mengen von 0,1 - 20, vorzugsweise 1 -10 und insbesondere 1- 5 Gew.-% - bezogen auf die Endformulierung der kosmetischen Zubereitung - eingesetzt.

### UV-Lichtschutzfilter

Überraschenderweise hat sich gezeigt, dass die Dimerdiole eine verbesserte Wasserfestigkeit von Sonnenschutzformulierungen bewirken und somit einen Langzeitschutz bei Aufenthalt im Wasser ermöglichen.

Da in 50 cm Wassertiefe immer noch ca. 60% UV-B- und ca. 80% UV-A-Strahlung (bezogen jeweils auf den UV-Anteil, der die Erdoberfläche erreicht) wirksam sind, ist die Wasserfestigkeit von Sonnenschutzmittel insbesondere für Kinder und Wassersportler besonders wichtig. Effektive Sonnenschutzmittel sollten wasserfest formuliert sein, gut auf der Haut haften und während des Aufenthaltes im Wasser nur langsam abgewaschen werden. Eine Sonnenschutzformulierung ist nach den Empfehlungen der COLIPA wasserfest, wenn nach einer definierten Wasserbelastung noch mindestens 50% der ursprünglichen Lichtschutzwirkung vorhanden ist. Die Lichtschutzwirkung wird hierbei durch Einsatz von geeigneten Lichtschutzfiltern erzielt.

UV-Lichtschutzfaktoren sind bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0 693 471 B1 beschrieben
➢ 4-Aminobenzoesäurederivate, vorzugsweise 4-(Dimethylamino)benzoesäure-2-ethyl-hexylester, 4-(Dimeihylamino)benzoesäure-2-octylester und 4-(Dimethylamino)benzoe-säureamylester
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene)
➢ Ester der Salicylsäure, vorzugsweise Salicylsäure-2-ethylhexylester, Salicylsäure-4-iso-propylbenzylester, Salicylsäurehomomenthylester
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
➢ Ester der Benzalmalonsäure, vorzugsweise 4-Methoxybenzmalonsäuredi-2-ethylhexyl-ester
➢ Triazinderivate, wie z.B. 2,4,6-Trianilino-(p-carbo-2'-ethyl-1'-hexyloxy)-1,3,5-triazin und Octyl Triazon, wie in der EP 0 818 450 A1 beschrieben oder Dioctyl Butamido Triazone (Uvasorb® HEB)
➢ Propan-1,3-dione, wie z.B. 1-(4-tert.Butylphenyl)-3-(4'methoxyphenyl)propan-1,3-dion
➢ Ketotricyclo(5.2.1.0)decan-Derivate, wie in der EP 0 694 521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze
➢ Sulfonsäurederivate von Benzophenonen, vorzugsweise 2-Hydroxy-4-methoxybenzo-phenon-5-sulfonsäure und ihre Salze
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische **UV-A-**Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 197 12 033 A1 (BASF). Die UV-A und UV-B-Filter können selbstverständlich auch in Mischungen eingesetzt werden. Erfindungsgemäß besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parf.Kosm. 3, 11 (1999**)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Kosmetische und/oder pharmazeutische Zubereitungen

Die erfindungsgemäß zu verwendenden Dimerdiole können in kosmetischen/pharmazeutischen Sonnenschutzmitteln, Zubereitungen der dekorativen Kosmetik, Insektenschutzmitteln oder Deo/Antitranspirant Formulierungen enthalten sein.

Diese Zubereitungen können als nahezu wasserfreie Öle, Cremes, Gele, Lotionen, alkoholische und wässrig/alkoholische Lösungen, Emulsionen, sprühbare Emulsionen, Wachs/Fett-Massen, Stiftpräparate, und dergleichen formuliert werden. Entsprechend weisen die Zusammensetzungen variierende Viskositäten von 100 - 1000000 mPa·s auf (Brookfield RVF, 23 °C, Spindel und Umdrehungen in Abhängigkeit von der Viskosität nach Angaben des Herstellers). Vorzugsweise weisen die Zubereitungen eine Viskosität von 100 - 500000 mPa·s bei 23 °C, und insbesondere von 1000 - 250000 mPa·s bei 23 °C auf. Je nach Applikationszweck sind eine Reihe weiterer Hilfs- und Zusatzstoffe enthalten, wie beispielweise Ölkörper, Emulgatoren, Tenside, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, Antioxidantien, Deodorantien, Antitranspirantien, Filmbildner, Quellmittel, Insektenrepellentien, Selbstbräuner, Tyrosininhibitoren, Hydrotrope, Solubilisatoren, Konservierungsmittel, Parfümöle, Farbstoffe und dergleichen.

### Ölkörper

Die Mittel enthalten vorzugsweise zusätzlich wenigstens einen Ölkörper. Unter Ölkörpern sind bei 20 °C flüssige, mit Wasser bei 25 °C nicht mischbare Stoffe oder Gemische von Stoffen zu verstehen. Die Kombination mit Ölkörpern erlaubt die Optimierung der sensorischen Eigenschaften der Zubereitungen. Je nach Applikationsform (z.B. Öl, Creme, Lotion, sprühbare Emulsion) kann die Menge der Ölkörper an der Gesamtzusammensetzung zwischen 1 und 98 Gew.-% variieren. Eine bevorzugte Ausführungsform der Zubereitungen enthält 1 - 30 Gew.-% Ölkörper, insbesondere 5 - 30 Gew.-% Ölkörper.

Als Ölkörper kommen beispielsweise Guerbetalkohole auf Basis von Fettalkoholen mit 6 bis 18, vorzugsweise 8 bis 10 Kohlenstoffatomen (z.B. Eutanol^{®} G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, lsostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen - insbesondere Diethylhexylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestem mit Polyolen (Hydagen^{®} HSP, Sovermol^{®} 750, Sovermol^{®} 1102), Siliconöle (Cyclomethicone, Siliciummethicontypen u.a.) und/oder aliphatische bzw. naphthenische Kohlenwasserstoffe, wie z.B. wie Mineralöl, Vaseline, Petrolatum, Squalan, Squalen oder Dialkylcyclohexane in Betracht.

Eine deutliche Verbesserung der sensorischen Eigenschaften der Zusammensetzungen ergibt sich, wenn Dialkylether und/oder Dialkylcarbonate als Olkörper eingesetzt oder mitverwendet werden daher sind diese als Ölkörper bevorzugt. Darüber hinaus kann es bevorzugt sein, Siliconverbindungen zu verwenden, um das unerwünschte, sogenannte "Weißeln" (Mikroschaumbildung) in kosmetischen Formulierungen zu verhindern. Beispielsweise werden Cyclomethicone und Dimethicone hierfür in Mengen von 1 - 20 Gew.% bezogen auf die Gesamtzusammensetzung eingesetzt.

### Tenside/Emulgatoren

Eine weitere bevorzugte Ausführungsform der Zubereitungen enthält zusätzlich wenigstens einen Emulgator. Der Zusatz von Emulgatoren verbessert die Einarbeitung der Dimerdiole.

Bevorzugt sind nicht-ionische Emulgatoren. Diese zeichnen sich durch ihre Hautfreundlichkeit und Milde sowie ihre ökotoxologisch guten Eigenschaften aus. Durch Verwendung einer Kombination nicht-ionischer W/O und O/W-Emulgatoren kann weiterhin Stabilität und Sensorik der Zusammensetzungen verbessert werden. Eine besonders bevorzugte Kombination ist unter der Bezeichnung Eumulgin^{®} VL 75 (Cognis Deutschland GmbH) im Handel. Die Zusammensetzungen enthalten den/die Emulgator(en) in einer Menge von üblicherweise 0,1 bis 15 Gew.-%, vorzugsweise 1 bis 10 Gew.-% und insbesondere 3 bis 10 Gew.-% bezogen auf das Gesamtgewicht der Zusammensetzung.

### Nicht-ionische Emulgatoren

Zur Gruppe der nicht-ionischen Emulgatoren gehören:
(1) Anlagerungsprodukte von 2 bis 50 Mol Ethylenoxid und/oder 0 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C_{12/18}-Fettsäuremono- und -diester von Anlagerungsprodukten von 1 bis 50 Mol Ethylenoxid an Glycerin,
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z.B. Polyolpoly-12-hydroxystearate, Polyglycerinpolyricinoleat, Polyglycerindüsostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose), oder Mischester wie z.B. Glycerylstearatcitrat und Glycerylstearatlactat.
(9) Wollwachsalkohole.
(10) Polysiloxan-Polyalkyl-Polyether-Copolymere bzw. entsprechende Derivate.
(11) Mischester aus Pentaerythrit, Fettsäuren, Citronensäure und Fettalkohol gemäß und/oder Mischester von Fettsäuren mit 6 bis 22 Kohlenstoffatomen, Methylglucose und Polyolen, vorzugsweise Glycerin oder Polyglycerin.
(12) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. Für die Zubereitungen sind die Umsetzungsprodukte mit 1 - 100 Mol Ethylenoxid besonders gut geeignet.

Erfindungsgemäß ebenso bevorzugt sind aufgrund ihrer Milde Polyolpoly-12-hydroxystearate und Abmischungen davon, welche beispielsweise unter den Marken "Dehymuls^{®} PGPH" (W/O-Emulgator) oder "Eumulgin^{®}VL 75° (Abmischung mit Coco Glucosides im Gewichtsverhältnis 1:1, O/W-Emulgator) oder Dehymuls^{®} SBL (W/O-Emulgator) von der Cognis Deutschland GmbH vertrieben werden. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefasst und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen-Gruppen, d.h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₃-C₆-Polyolen, wie beispielsweise Glycerylmonoestem, Partialester des Pentaerythrits oder Zuckerestem, z.B. Saccharosedistearat, Sorbitanmonoisostearat, Sorbitansesquüsostearat, Sorbitandiisostearat, Sorbitantrüsostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Im Falle der Einarbeitung wasserlöslicher Wirkstoffe und Wasser sollte wenigstens ein Emulgator aus der Gruppe nicht-ionischer O/W-Emulgatoren (HLB-Wert: 8-18) und/oder Solubilisatoren eingesetzt werden. Hierbei handelt es sich beispielsweise um die bereits einleitend erwähnten EthylenoxidAddukte mit einem entsprechend hohen Ethoxylierungsgrad, z.B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-20 und PEG-20 Glyceryl Stearate.

Nicht-ionische Emulgatoren aus der Gruppe der Alkyloligoglycoside sind besonders hautfreundlich und daher im Sinne der Erfindung bevorzugt als O/W-Emulgatoren geeignet. Sie erlauben eine Optimierung der sensorischen Eigenschaften der Zusammensetzungen und erlauben eine besonders leichte Einarbeitbarkeit der Dimerdiole. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen, vorzugsweise 12 bis 22, und besonders bevorzugt 12 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Eine Zusammensetzung, in der die Dimerdiole erfindungsgemäß verwendet werden kônnen enthält:
(a) 1-10 Gew.-%, vorzugsweise 1- 5% Gew.-%, Dimerdiole
(b) 1,0 - 30,0 Gew.-% UV-Lichtschutzfilter
(c) 5 - 30 Gew.-% Ölkörper
(d) 0,1-10 Gew.-% Emulgator(en)
(e) 0 - 90 Gew.-% Wasser.

### Weitere Tenside/Emulgatoren

Die Zusammensetzungen können je nach Verwendungszweck weiterhin zwitterionische, amphotere, kationische und ferner anionische Tenside enthalten. Bevorzugt geeignet ist auch die Kombination mit ausgewählten anionischen Tensiden (vide infra).

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl-oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

In einer weiteren bevorzugten Ausführungsform enthalten die Zubereitungen wenigstens einen Emulgator ausgewählt aus der Gruppe der anionischen Emulgatoren, insbesondere der Alkylphosphate und Alkylsulfate. Anionische Emulagtoren/Tenside tragen zu einer weiteren Verbesserung der Wasserfestigkeit der Zubereitungen bei.

Anionische Tenside sind gekennzeichnet durch eine wasserlöslich machende, anionische Gruppe wie z.B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C- Atomen sowie Suitesuccinate und Acylglutamate in Form ihrer Alkali- oder Ammoniumsalze. Für die erfindungsgemäßen Zubereitungen sind unter den anionischen Tensiden Alkalisalze von Fettsäuren (Natriumstearat) und insbesondere Alkylsulfate (Lanette^{®} E) sowie Alkylphosphate (Amphisol^{®} K) bevorzugt geeignet,da sie zu besonders stabilen und homogenen Emulsionen mit höheren Viskositäten führen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie verleihen den Zusammensetzungen einen besonderen Weichgriff. Es handelt sich dabei um bekannte Stoffe, die man nach den einschlägigen Methoden der organischen Chemie herstellt. Weitere erfindungsgemäß verwendbare kationische Tenside stellen die quatemisierten Proteinhydrolysate dar.

### Feuchthaltemittel/Hautbefeuchtungsmittel

In einer bevorzugten Ausführungsform enthält die Zusammensetzung auch ein Feuchthaltemittel. Dieses dient zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 - 15 Gew.-%, vorzugsweise 1-10 Gew.-%, und insbesondere 5 - 10 Gew.-% enthalten.

Geeignet sind u.a. Aminosäuren, Pyrrolidoncarbonsäure, Milchsäure und deren Salze, Lactitol, Hamstoff und Hamstoffderivate, Hamsäure, Glucosamin, Kreatinin, Spaltprodukte des Kollagens, Chitosan oder Chitosansalze/-derivate, und insbesondere Polyole und Polyolderivate (z.B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG.10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20; Sorbeth-30, Sorbeth-40), Honig und gehärteter Honig, gehärtete Stärkehydrolysate sowie Mischungen aus gehärtetem Weizenprotein und PEG-20-Acetatcopolymer. Bevorzugt geeignet als Feuchthaltemittel sind Glycerin, Diglycerin, Triglycerin und Butylenglycol.

### Viskositätsregulatoren

Die gewünschte Viskosität der Zusammensetzungen wird durch Zugabe von Viskositätsregulatoren erreicht. Viskositätsregulatoren erhöhen zusätzlich die Wasserfestigkeit der Zubereitungen. Eine bevorzugte Ausführungsform der Zubereitung enthält daher zusätzlich wenigstens einen Viskositätsregulator. Als Viskositätsregulatoren kommen u.a. Konsistenzgeber, wie z.B. Fettalkohole oder Hydroxyfettalkohole mit 12 bis 22 und vorzugsweise 16 bis 18 Kohlenstoffatomen sowie Partialglyceride, Fettsäuren mit 12 bis 22 Kohlenstoffatomen oder 12-Hydroxyfettsäuren in Betracht. Bevorzugt geeignet ist eine Kombination dieser Stoffe mit Alkyloligoglucosiden und/oder Fettsäure-N-methylglucamiden gleicher Kettenlänge und/oder Polyglycerinpoly-12-hydroxystearaten,da derartige Kombinationen besonders stabile und homogene Emulsionen liefern. Zu den Viskositätsregulatoren zählen auch Verdickungsmittel wie beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), unvernetzte und mit Polyolen vernetzte Polyacrylsäuren, Polyacrylamide, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Auch Tenside, wie beispielsweise ethoxylierte Fettsäureglyceride, Ester von Fettsäuren mit Polyolen, wie beispielsweise Pentaerythrit oder Trimethylolpropan, Fettalkoholethoxylate mit eingeengter Homologenverteilung, Alkyloligoglucoside sowie Elektrolyte, wie z.B. Kochsalz und Ammoniumchlorid können zur Viskositätsregulierung eingesetzt werden.

Als Viskositätsregulatoren eignen sich auch anionische, zwitterionische, amphotere und nichtionische Copolymere wie beispielsweise Vinylacetat/Crotonsäure-Copolymere, VinylpyrrolidonNinylacrylat-Copolymere, Vinylacetat/Butylmaleat/lsobomylacrylat-Copolymere, Methylvinylether/Maleinsäureanhydrid-Copolymere und deren Ester, Acrylamidopropyltrimethylammoniumchlorid/Acrylat-Copolymere, Octylacrylamid/Methylmethacrylat/tert.-Butylaminoethylmethacrylat/2-Hydroxypropylmethacrylat-Cop6lymere, Vinylpyrrolidon/Vinylacetat-Copolymere, Vinylpyrrolidon/Dimethylaminoethylmethacrylat/Vinlcaprolactam-Terpolymere sowie gegebenenfalls derivatisierte Celluloseether und Silicone. Weitere geeignete Polymere und Verdickungsmittel sind in Cosm.Toil. 108, 95 (1993**)** aufgeführt.

Bevorzugt ist der Einsatz von Polymeren in Mengen von 0,1 - 5 Gew.-%, vorzugsweise 0,1 - 3 Gew.-% und insbesondere 0,1 - 2 Gew.-% bezogen auf die Gesamtzusammensetzung. Polyacrylsäure-Homo- und Copolymere sind bevorzugt geeignet, da die resultierenden Zubereitungen auch bei Langzeitlagerung unter Temperaturbelastung kaum oder keine Viskositätsschwankungen zeigen.

### lnsektenrepellent-Wirkstoffe

Eine weitere bevorzugte Ausführungsform der Zusammensetzung enthält zusätzlich wenigstens einen Insektenrepellent-Wirkstoff oder eine Kombination dieser Wirkstoffe. Auch für diese Applikationsform, ist die Wasserfestigkeit der Mittel wichtig, damit die Wirkstoffe möglichst nicht abgewaschen werden und sich ein Langzeitschutz entfaltet. Die Kombination von UV-Filtern und Insektenrepellent-Wirkstoffen in einem Mittel ist auch aus Kostengründen besonders lukrativ.

Als **Insekten-Repellentien** kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent 3535 von der Merck KGaA vertrieben wird, sowie Butylacetylaminopropionate in Frage. Sie werden in den Zusammensetzungen üblicherweise in einer Menge von 0,1 - 10 Gew.-%, vorzugsweise 1 - 8 Gew.-%, und besonders bevorzugt in einer Menge von 2 - 6 Gew.-% bezogen auf die Gesamtzusammensetzung eingesetzt.

### Weitere Hilfs- und Zusatzstoffe (fakultativ)

Die Zusammensetzungen können je nach Art und Zweck der Applikation weitere Hilfs- und Zusatzstoffe enthalten, wie beispielsweise Fette und Wachse, Periglanzwachse, Überfettungsmittel, Stabilisatoren, kationische, zwitterionische oder amphotere Polymere, biogene Wirkstoffe, Filmbildner, Quellmittel, Hydrotrope, Konservierungsmittel, Antischuppenmittel, Selbstbräuner, Solubilisatoren, Parfümöle, Farbstoffe, etc., die nachstehend beispielhaft aufgeführt sind.

### Deo- und Antitrensnirant-Wirkstoffe

Es kann wünschenswert sein, Sonnenschutzmitteln Deo- und Antitranspirantwirkstoffe zuzusetzen, um beispielsweise im Gesichtsbereich die Transpiration einzuschränken oder zu unterdrücken.

Zu diesen Wirkstoffen gehören adstringierende Metallsalze (antitranspirante Wirkstoffe), keimhemmende Mittel, Enzyminhibitoren, Geruchsabsorber, Geruchsüberdecker oder eine beliebige Kombination dieser Wirkstoffe. Die Deo-/Antitranspirant-Wirkstoffe können in den Zusammensetzungen in einer Menge von 0,1 - 30 Gew.-%, vorzugsweise 5 - 25 Gew.-% und insbesondere 10 - 25 Gew.-% enthalten sein (bezogen auf die Menge der Aktivsubstanz).

Als **Antitranspirant-Wirkstoffe** kommen z.B. Aluminiumchlorhydrate, Aluminium-Zirkonium-Chlorohydrate sowie Zinksalze in Frage. Diese wirken wahrscheinlich über den partiellen Verschluss der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron^{®} der Clariant GmbH, befindet beispielsweise sich ein Aluminiumchlorhydrat im Handel, das der Formel [Al₂(OH)₅Cl]·2,5 H₂O entspricht, und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal^{®} 36G vermarktet werden.

Als weitere Deowirkstoffe können Enzyminhibitoren beispielsweise Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} C.A.T., Cognis Deutschland GmbH). Die Stoffe inhibieren die Enzymaktivität von schweißzersetzenden Bakterien und reduzieren dadurch die Geruchsbildung. Wahrscheinlich wird dabei durch die Spaltung des Citronensäureesters die freie Säure freigesetzt, die den pH-Wert auf der Haut soweit absenkt, dass dadurch die Enzyme inhibiert werden. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Apfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Zusammensetzungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan^{®} von der Ciba-Geigy, Basel/CH vertrieben wird.

Als **keimhemmende Mittel** sind grundsätzlich alle gegen gram-positive Bakterien wirksamen Stoffe geeignet, wie z.B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z.B. Salicylsäure-n-octylamid oder Salicylsäure-n-decylamid.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z.B. Extrakte von Labdanum bzw. Styrax oder bestimmte Abietinsäurederivate.

Als **Geruchsüberdecker** fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Unter **Fetten und Wachsen** werden im Sinne der Erfindung alle Lipide mit fett- oder wachsartiger Konsistenz verstanden, die einen Schmelzpunkt oberhalb von 20 °C aufweisen. Hierzu gehören beispielsweise die klassischen Triacylglycerine, also die Dreifachester von Fettsäuren mit Glycerin, die pflanzlicher oder tierischer Herkunft sein können. Hierbei kann es sich auch um Mischester, also um Dreifachester aus Glycerin mit verschiedenen Fettsäuren, oder aber um ein Gemisch verschiedener Glyceride handeln. Hierzu gehören auch Gemische aus Mono- Di- und Triglyceriden. Erfindungsgemäß besonders gut geeignet sind sogenannte gehärtete Fette und Öle, die durch Partialhydrierung gewonnen werden. Pflanzliche gehärtete Fette und Öle sind bevorzugt, z.B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett. Besonders geeignet sind oxidationsstabile pflanzliche Glyceride, die unter der Bezeichnung Cegesoft^{®} oder Novata^{®} angeboten werden.

Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Neben den Fetten kommen als Zusatzstoffe auch fettähnliche Substanzen, wie Lecithine und Phospholipide in Frage. Lecithine sind Glycero-Phospholipide, die sich aus Fettsäuren, Glycerin, Phosphorsäure und Cholin durch Veresterung bilden, und häufig auch als Phosphatidylcholine (PC) bezeichnet werden. Als Beispiel für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate). Auch Sphingosine bzw. Sphingolipide kommen als fettartige Stoffe in Frage.

Als **Perlglanzwachse** geeignet sind beispielsweise Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit C₆-C₂₂-Fettalkoholen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen - speziell Lauron^{®} ; Distearylether; Fettsäuren wie Stearinsäure, C₁₂-C₂₂-Hydroxyfettsäuren, Behensäure, Ringöffnungsprodukte von C₁₂-C₂₂-Olefinepoxiden mit C₁₂-C₂₂-Fettalkoholen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei letztere gleichzeitig als Schaumstabilisatoren dienen.

Als sogennnte **Stabilisatoren** können Metallsalze von Fettsäuren, wie z.B. Magnesium-, Aluminium-und/oder Zinkstearat bzw. -ricinoleat eingesetzt werden.

Geeignete **kationische Polymere**, welche die Sensorik der erfindungsgemäßen Zusammensetzungen weiter optimieren und der Haut ein Gefühl der Weichheit verleihen, sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Geeignete **Siliconverbindungen** wurden bereits bei den Ölkörpern erwähnt. Neben Dimethylpolysiloxanen, Methylphenylpolysiloxanen und cyclischen Siliconen sind auch amino-, fettsäure-, alkohol-, polyether-, epoxy-, fluor-, glykosid- und/oder alkylmodifizierte Siliconverbindungen geeignet, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und Siliciumdioxid oder hydrierten Silicaten handelt.

Erfindungsgemäß geeignete **biogene Wirkstoffe** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen. Derartige Wirkstoffe werden als Radikalfänger in Sonnenschutzformulierungen eingesetzt und dienen der Regeneration der Haut.

**Sogenannte Filmbildner**, die zu einer weiteren Verbesserung der Sensorik der erfindungsgemäßen Zubereitungen führen, sind beispielsweise Chitosan, mikrokristallines Chitosan, quaterniertes Chitosan, Kollagen, Hyaluronsäure bzw. deren Salze und ähnliche Verbindungen, sowie die bereits unter den Viskositätsregulatoren genannten Polyvinylpyrrolidone, Vinylpyrrolidon-Vinylacetat-Copolymerisate, Polymere der Acrylsäurereihe und quaternäre Cellulose-Derivate.

Als **Antischuppenwirkstoffe** kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2,4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyethylenglykolsorbitanmonooleat, Schwefelrizinolpolyethoxylat, Schwefelteer-Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion/Dipyrithion-Magnesiumsulfat in Frage.

Als **Selbstbräuner** eignet sich z.B. Dihydroxyaceton. Als **Tyrosinaseinhibltoren**, welche die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Zur Verbesserung des Fließverhaltens der Zusammensetzungen können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
➢ Glycerin
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
➢ technische Oligoglyceringemische mit einem Eigenkondensationsgrad von 1,5 bis 10 wie etwa technische Diglyceringemische mit einem Diglyceringehalt von 40 bis 50 Gew.-%
➢ Methyolverbindungen, wie insbesondere Trimethylolethan, Trimethylolpropan, Trimethylolbutan, Pentaerythrit und Dipentaerythrit
➢ Kurzkettige Alkyglucoside, insbesondere solche mit 1 bis 8 Kohlenstoffen im Alkylrest, wie beispielsweise Methyl- und Butylglucosid
➢ Zuckeralkohole mit 5 bis 12 Kohlenstoffatomen, wie beispielsweise Sorbit oder Mannit
➢ Zucker mit 5 bis 12 Kohlenstoffatomen, wie beispielsweise Glucose oder Saccharose
➢ Aminozucker, wie beispielsweise Glucamin
➢ Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt natürliche, pflanzliche und tierische sowie synthetische Riechstoffe oder deren Gemische. Natürliche Riechstoffe werden u.a. durch Extraktion von Blüten, Stengeln, Blättern, Früchten, Fruchtschalen, Wurzeln und Harzen von Pflanzen erhalten. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Bevorzugt werden Mischungen verschiedener Riechstoffe verwendet, die gemeinsam eine ansprechende Duftnote erzeugen.

Als **Farbstoffe** können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind.

Der Gesamtanteil der Hilfs- und Zusatzstoffe kann 1 bis 50, vorzugsweise 5 bis 40 Gew.-% - bezogen auf die Zubereitungen betragen. Die Herstellung der Zubereitungen erfolgt durch übliche Kalt - oder Heißprozesse; vorzugsweise arbeitet man nach der Phaseninversionstemperatur-Methode.

### Erfindungsgemäße Beispiel

Sovermol^{®} 908 (Cognis Deutschland GmbH) wurde in Basisrezepturen eingearbeitet und die Wasserfestigkeit der Mittel bestimmt. Zur Bestimmung der Wasserfestigkeit der Mittel wurde eine definierte Menge der Zubereitungen (gemäß Tabelle 1) auf ein geeignetes Trägermaterial aufgetragen und in einem Becherglas nach vorgegebenen Kriterien gewässert, wobei mittels eines Magnetrührers das Wasser in Bewegung gehalten wurde. Der SPF (Sun protection factor) wurde vor und nach dem Wässern mit dem UV-1000S Labsphere Ultraviolet Transmittance Analyser bestimmt.

Die sensorische Beurteilung erfolgte durch ein Panel zehn geschulter Probanden, die Noten von (1) = sehr gut bis (6) = ungenügend, vergaben. Angegeben sind Mittelwerte aus jeweils drei Messungen.

### Wasserfestigkeit:

➢ Trägermaterial: Vitro-Skin N19, Firma IMS (4 x 3 cm) auf Diarahmen
➢ Auftragsmenge: 2 mg/cm²
➢ Trockenzeit vor der 1. Messung: 15 min, Temperatur 30°C
➢ Wassertemperatur: 23°C (16 °d)
➢ pH-Wert Wasser: 7,0 +/- 0,5
➢ Wassermenge: 400 ml
➢ Rührgeschwindigkeit: 300 UpM (Magnetrührer)
➢ Wässerungszeit: 2 x 20 min mit einer Pause von 20 min
➢ Trockenzeit vor der 2. Messung: 15 min, Temperatur 30 °C

Die Ergebnisse sind in Tabelle 1 zusammengefasst. Die Beispiele 1 und 2 sind erfindungsgemäß, die Beispiele V1 und V2 dienen zum Vergleich. Die Mengenangaben in nachfolgenden Beispielen beziehen sich, soweit nicht anders angegeben, auf Gew.-% der handelsüblichen Substanzen in der Gesamtzusammensetzung.

**Tabelle 1: Basisrezepturen von Sonnenschutzformulierungen; Wasserfestigkeit und Sensorik**

| **Zusammensetzung / Performance** | **1** | **2** | **V1** | **V2** |
|---|---|---|---|---|
| Eumulgin^{®} VL 75 | 4.0 | 4.0 | 4.0 | 4.0 |
| Myritol^{®} 331 | 7.0 | 7.0 | 7.0 | 7.0 |
| Cetiol^{®} OE | 6.0 | 6.0 | 6.0 | 6.0 |
| Eutanol^{®} G 16 | 3.0 | 3.0 | 3.0 | 3.0 |
| Sovermol^{®} 908 | 4.0 | 2.0 | - | - |
| Antaron^{®} V 220 | | 2.0 | 4.0 | |
| Antaron^{®} V 216 | | - | | 4.0 |
| Neo Heliopan^{®} AV | 7.5 | 7.5 | 7.5 | 7.5 |
| Parsol^{®} 1789 | 2.0 | 2.0 | 2.0 | 2.0 |
| Carbopol^{®} 2984 | 0.2 | 0.2 | 0.2 | 0.2 |
| Glycerin | 5.0 | 5.0 | 5.0 | 5.0 |
| Wasser, NaOH, Konservierungsmittel | ad 100 / pH = 7 / q.s. | | | |

| ***In-vitro Sun-Protection-Factor (SPF)*** | | | | |
|---|---|---|---|---|
| *- vor der Wasserbehandlung* | 15 | 15 | 15 | 15 |
| *- nach der Wasserbehandlung* | 14 | 13 | 9 | 10 |
| *- Differenz (%-rel.)* | 93 | 87 | 60 | 66 |

| ***Sensorische Beurteilung*** | | | | |
|---|---|---|---|---|
| *Einziehvermögen* | 1 | 2 | 5 | 4 |
| *Glätte* | 1 | 2 | 4 | 4 |
| *Klebrigkeit* | 1 | 1 | 6 | 5 |

Die Vergleichsbeispiele V1 und V2, die anstelle von Sovermol^{®} 908 Antaron^{®} V 220 und Antaron^{®} V 216 enthalten (diese werden üblicherweise eingesetzt, um die Wasserfestigkeit zu erhöhen), zeigen eine deutlich reduzierte Wasserfestigkeit und werden hinsichtlich sensorischer Gesichtspunkte als minderwertiger eingestuft.

**Tabelle 2: O/W-Sonnenschutzemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **3** | **4** | **5** | **6** | **7** | **8** | **9** | **10** | **11** | **12** | **13** |
|---|---|---|---|---|---|---|---|---|---|---|---|
| **L = Lotion, C = Creme, S = Spray** | L | C | S | L | C | L | L | C | L | C | L |
| Eumulgin^{®} VL 75 | | | | | | 4 | 4 | 2 | | | 2 |
| Eumulgin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®} O | 1 | 6 | | | 5 | 2 | | 2 | | | |
| **Sovermol^{®} 908** | 2 | 2 | 4 | 1 | 2 | 2 | 2 | 3 | 2 | 2 | 1 |
| Emery^{®} 1780 | | | | | 0.5 | 0.5 | | | | | |
| Lanolin, wasserfrei USP | | | | | | | 5 | | | | |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | 3 |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} Hydro (Na-Salz) | 2 | | 2.2 | | 3 | 3 | | | | | 2 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | 2 | 2.5 | | | 1 | 1.5 | 2 | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.75 | | | | | 1 | 1 | | |
| Keitrol^{®} T | | | 0.25 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH Wasser | q.s. / pH 6.5 - 7.5 / ad 100 | | | | | | | | | | |

**Tabelle 3: O/W-Sonnenschutzemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **14** | **15** | **16** | **17** | **18** | **19** | **20** | **21** | **22** | **23** | **24** |
|---|---|---|---|---|---|---|---|---|---|---|---|
| **L = Lotion, C = Creme, S = Spray** | L | L | L | C | L | C | S | C | C | L | L |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Myrj^{®} 51 | | | | | | | | | | | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} 0 | | | | 2 | | | | | 1 | 1 | |
| Sovermol^{®} 908 | 4 | 2 | 4 | 1 | 2 | 4 | 2 | 2 | 2 | 4 | 3 |
| Emery^{®} 1780 | | | | | | | | | | | |
| Lanolin, wasserfrei, USP | | | | | | | | | | | |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow-Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | 3 | 4 | | 7 |
| Neo Heliopan^{®} BB | | | | | | 1 | | 1 | 1 | | 1 |
| Neo Heliopan^{®} MBC | | 2 | | 1 | | 2 | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | 3 | | 5 | 4 | 7.5 | 3 |
| Uvinul^{®} T 150 | 1 | | 1 | | 1 | 1 | 1 | | 1.3 | 1 | 1 |
| Parsol^{®} 1789 | 1 | | | | | 1 | | | 2 | | 1 |
| Z-Cote^{®} HP 1 | 7 | 2 | 5 | | | 7 | 5 | | 6 | 2 | 2 |
| Eusolex^{®} T 2000 | 5 | 2 | | | 10 | | | 10 | | 2 | 2 |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100 / q.s. / pH 6.5 -7.5 | | | | | | | | | | |

**Tabelle 4: W/O-Sonnenschutzemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **26** | **27** | **28** | **29** | **30** | **31** | **32** | **33** | **34** | **35** | **36** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Microkristallines Wachs | | | 5 2 | | | | | | | | 5 |
| Bienenwachs | 1 | | | 1 | | | | 5 | | 7 | |
| Tego^{®} Care CG | | | | | 1 | | | | | | .5 |
| Prisorine^{®} 3505 | 1 | | 1 | 1 | | 1 | 1 | | | | 1 |
| **Sovermol^{®} 908** | **3** | **4** | **2** | **4** | **4** | **2** | **2** | **2** | **1** | **1** | **3** |
| Emery^{®} 1780 | | | 5 | | | | | | | 4 | |
| Wollwachsalkohol, wasserfrei, USP | | | | | | | | | | | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G | | | 3 | | | | | 8 | | | |
| Cetiol^{®} PGL | | 11 | | | | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 2 | | 3 | 1 | | | 2 | 1 | 1 | |
| Neo Heliopan^{®} 303 | | | | 2 | 4 | | | 2 | | 6 | |
| Neo Heliopan^{®} BB | | 4 | 2 | | | | 2 | | | | |
| Neo Heliopan^{®} MBC | | | | 2 | 2 | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | 3 | 7.5 | 7.5 | | 5 | | 7.5 |
| Uvinul^{®} T 150 | | 1 | | 2 | 2.5 | | | 1 | | 2 | |
| Parsol^{®} 1789 | | 2 | | | 1 | | | 1 | | 2 | |
| Zinkoxid NDM | | | | 2 | 5 | 6 | 4 | | 3 | 2 | 15 |
| Eusolex^{®} T 2000 | 15 | | 10 | 2 | 5 | 6 | 4 | | 3 | 2 | |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser / Konservierungsmittel | ad 100 / q.s. | | | | | | | | | | |

**Tabelle 5: W/O-Sonnenschutzemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **37** | **38** | **39** | **40** | **41** | **42** | **43** | **44** | **45** | **46** | **47** |
|---|---|---|---|---|---|---|---|---|---|---|---|
| **L = Lotion; C = Creme** | L | C | L | L | C | L | L | L | L | C | C |
| Dehymuls^{®} PGPH | 3 | 1 | 5 | 1 | 1 | 3 | 2 | 4 | 0.5 | 1 | 4 |
| Monomuls^{®} 90-018 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Mikrokristallines Wachs | | | | | 4 | | 1 | | | 4 | |
| Bienenwachs | | 4 | | 2 | | | 1 | | 2 | | 1 |
| Tego^{®} Care CG | | | | | | | | | | | |
| Isostearinsäure | 1 | 1 | | | | | 1 | 1 | | 1 | 1 |
| **Sovermol^{®} 908** | 2 | 4 | 3 | 3 | 2 | 2 | 1 | 3 | 3 | 1 | 4 |
| Emery^{®} 1780 | | 7 | 3 | | | | | | | | |
| Wollwachsalkohol, wasserfrei, USP | | | | | | | | | | | |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Coming DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G | | | | 2 | | | | | 5 | | |
| Cetiol^{®} PGL | | | | | | | | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | 1 | 1 | | | 4 | | | |
| Neo Heliopan^{®} 303 | 6 | 2 | | | 2 | | | | 6 | | |
| Neo Heliopan^{®} BB | | 2 | | 2 | | 2 | | | | | |
| Neo Heliopan^{®} MBC | 2 | | | 1 | 3 | | 4 | | 2 | | |
| Neo Heliopan^{®} OS | | | | | 4 | | 8 | | | | |
| Neo Heliopan^{®} E 1000 | | | 5 | | | | | | | 5 | |
| Neo Heliopan^{®} AV | | 5 | 5 | 6 | 4 | 7.5 | | | | 5 | |
| Uvinul^{®} T 150 | 1 | 1 | | 2 | 2 | | | | 3 | 2 | |
| Parsol^{®} 1789 | | 1 | 1 | 1 | | | 1 | | 0.5 | | |
| Z-Cote^{®} HP 1 | 4 | 10 | | 2 | 2 | | 8 | 5 | 5 | | 5 |
| Titandioxid T 805 | | | | 2 | 2 | 10 | | 7 | 7 | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser / Konservierungsmittel | ad 100 / q.s. | | | | | | | | | | |

**Tabelle 6: W/O-Pflegeemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **48** | **49** | **50** | **51** | **52** | **53** | **54** | **55** | **56** | **57** | **58** |
|---|---|---|---|---|---|---|---|---|---|---|---|
| **L = Lotion. C = Creme** | C | L | C | L | C | L | L | L | C | C | C |
| **Sovermol^{®} 908** | 1 | 1 | 2 | 1 | 1 | 1 | 1 | 1 | 2 | 2 | 1 |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | 2 | | 5 |
| Cetiol^{®} SN | | 5 | | | | | | 3 | | | |
| Cetiol^{®} OE | 3 | | | | 4 | | 2 | | 4 | 2 | |
| Dow Coming DC^{®} 244 | | | | | 1 | | 2 | | | | |
| Dow Coming DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser / Konservierungsmittel | Ad 100 / q.s. | | | | | | | | | | |

**Tabelle 7: W/O-Pflegeemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **59** | **60** | **61** | **62** | **63** | **64** | **65** | **66** | **67** | **68** | **69** |
|---|---|---|---|---|---|---|---|---|---|---|---|
| **L = Lotion. C = Creme** | L | C | L | L | C | L | L | L | L | C | C |
| Sovermol^{®} 908 | 1 | 3 | 1 | 2 | 1 | 2 | 1 | 1 | 1 | 1 | 3 |
| Dehymuls^{®} PGPH | 3 | 1 | 5 | 1 | 1 | 3 | 3 | 4 | 1 | 1 | 1 |
| Monomuls^{®} 90-018 | | 1 | | 1 | | | | | | | |
| Lameform^{®} TGI | | | | | 4 | | | 1 | | 3 | |
| Abil^{®} EM 90 | | | | 3 | | | | 1 | | 2 | |
| Isolan^{®} PDI | | 3 | | | | | | | | | 4 |
| Glucate^{®} DO | 1 | | | | | | | | | | |
| Arlace^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Mikrokristallines Wachs | | | | | 4 | | 1 | | | 4 | |
| Bienenwachs | | 4 | | 2 | | 2 | 1 | 1 | 2 | | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning ^{®} DC 244 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G 16 | 1 | | | | | | | | | | |
| Eutanol^{®} G | | | | 2 | | | | | 5 | | |
| Cetiol^{®} PGL | | | 10 | | | | | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser / Konservierungsmittel | ad 100 / q.s. | | | | | | | | | | |

**Tabelle 8: O/W-Pflegeemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **70** | **71** | **72** | **73** | **74** | **75** | **76** | **77** | **78** | **79** | **80** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| **Sovermol^{®} 908** | 2 | 2 | 1 | 1 | 1 | 1 | 2 | 3 | 2 | 2 | 1 |
| Eumulgin^{®} VL 75 | | | | | | 4 | 2 | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 5 | | | | |
| Cetiol^{®} SB 45 | | | 1.5 | | | | 2 | | | | |
| Cegesoft^{®} C 17 | | | | | | | | | | | |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | | | | | | 4 | 3 | |
| Dow Corning DC^{®} 245 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | | | 2 | |
| Eutanol^{®} G | | 2 3 | | | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | 2 | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | 0.1 | 0.3 | | | |
| Carbopol^{®} Ultrez 10 | | 0.3 | 0.3 | 0.2 | 0.2 | 0.2 | | | 0.1 | 0.3 | 0.2 |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100 / q.s. / pH 6.5 - 7.5 | | | | | | | | | | |

**Tabelle 9: O/W-Pflegeemulsionen**

| | | | | | | | | | | | |
|---|---|---|---|---|---|---|---|---|---|---|---|
| Mengenangaben beziehen sich auf Gew.-% der handelsüblichen Substanzen in der Gesamtzusammensetzung. | | | | | | | | | | | |

| **Komponente** | **81** | **82** | **83** | **84** | **85** | **86** | **87** | **88** | **89** | **90** | **91** |
|---|---|---|---|---|---|---|---|---|---|---|---|
| **L = Lotion, C = Creme** | C | C | L | C | L | C | L | L | L | L | C |
| **Sovermol^{®} 908** | 2 | 3 | 1 | 2 | 1 | 2 | 1 | 1 | 1 | 1 | 3 |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | 1 | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Novata^{®} AB | | | | | | | | | | | |
| Emery^{®} 1780 | | | | | | | | | | | |
| Lanolin, wasserfrei, USP | | | | | | 4 | | | | | |
| Cetiol^{®} SB 45 | | | | | | | 2 | | | | |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Coming DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser / Konservierungsmittel / NaOH | ad 100 / q.s. / pH 6.5 - 7.5 | | | | | | | | | | |

### Anhang

1) Abil^{®} EM 90
   INCI: Cetyl Dimethicone Copolyol
   Hersteller: Tego Cosmetics (Goldschmidt)
2) Amphisol^{®} K
   INCI: Potassium Cetyl Phosphate
   Hersteller: Hoffmann La Roche
3) Antaron^{®} V 220
   INCI: PVP/Eicosene Copolymer
   Hersteller: GAF General Aniline Firm Corp. (IPS-Global)
4) Antaron^{®} V 216
   INCI: PVP/Hexadecene Copolymer
   Hersteller: GAF General Aniline Firm Corp. (IPS-Global)
5) Arlacel^{®} 83
   INCI: Sorbitan Sesquioleate
   Hersteller: Uniqema (ICI Surfacants)
6) Arlacel^{®} P 135
   INCI: PEG-30 Dipolyhydroxystearate
   Hersteller: Uniqema (ICI Surfacants)
7) Bentone^{®} 38
   INCI: Quaternium-18 Hectorite
   Hersteller: Rheox (Elementis Specialties)
8) Carbopol^{®} 980
   INCI: Carbomer
   Hersteller: Goodrich
9) Carbopol^{®} 2984
   INCI: Carbomer
   Hersteller: Goodrich
10) Carbopol^{®} ETD 2001
   INCI: Carbomer
   Hersteller: BF Goodrich
11) Carbopol^{®} Ultrez 10
   INCI: Carbomer
   Hersteller: Goodrich
12) Cegesoft^{®} C 17
   INCI: Myristyl Lactate
   Hersteller: Cognis Deutschland GmbH, Grünau
13) Ceraphyl^{®} 45
   INCI: Diethylhexyl Malate
   Hersteller: International Specialty Products
14) Cetiol^{®} 868
   INCI: Ethylhexyl Stearate
   Hersteller: Cognis Deutschland GmbH
15) Cetiol^{®} A
   INCI: Hexyl Laurate
   Hersteller: Cognis Deutschland GmbH
16) Cetiol^{®} B
   INCI: Dibutyl Adipate
   Hersteller: Cognis Deutschland GmbH
17) Cetiol^{®} J 600
   INCI: Oleyl Erucate
   Hersteller: Cognis Deutschland GmbH
18) Cetiol^{®} OE
   INCI: Dicaprylyl Ether
   Hersteller: Cognis Deutschland GmbH
19) Cetiol^{®} PGL
   INCI: Hexyldecanol, Hexyldecyl Laurate
   Hersteller: Cognis Deutschland GmbH
20) Cetiol^{®} CC
   INCI: Dicaprylyl Carbonate
   Hersteller: Cognis Deutschland GmbH
21) Cetiol^{®} SB 45
   INCI: Shea Butter Butyrospermum Parkii (Linne) Hersteller: Cognis Deutschland GmbH
22) Cetiol^{®} SN
   INCI: Cetearyl Isononanoate
   Hersteller: Cognis Deutschland GmbH
23) Cutina^{®} E 24
   INCI: PEG-20 Glyceryl Stearate
   Hersteller: Cognis Deutschland GmbH
24) Cutina^{®} MD
   INCI: Glyceryl Stearate
   Hersteller: Cognis Deutschland GmbH
25) Dehymuls^{®} FCE
   INCI: Dicocoyl Pentaerythrityl Distearyl Citrate
   Hersteller: Cognis Deutschland GmbH
26) Dehymuls^{®} HRE 7
   INCI: PEG-7 Hydrogenated Castor Oil
   Hersteller: Cognis Deutschland GmbH
27) Dehymuls^{®} PGPH
   INCI: Polyglyceryl-2 Dipolyhydroxystearate
   Hersteller: Cognis Deutschland GmbH
28) Dow Corning^{®}244 Fluid
   INCI: Cyclomethicone
   Hersteller: Dow Coming
29) Dow Corning^{®} 245 Fluid
   INCI: Cyclopentasiloxane Cyclomethicone
   Hersteller: Dow Corning
30) Dow Corning^{®} 2502
   INCI: Cetyl Dimethicone
   Hersteller: Dow Corning
31) Dry^{®}Flo Plus
   INCI: Aluminium Starch Octenylsuccinate
   Hersteller: National Starch
32) Elfacos^{®}ST 37
   INCI: PEG-22 Dodecyl Glycol Copolymer
   Hersteller: Akzo-Nobel
33) Elfacos^{®}ST 9
   INCI: PEG-45 Dodecyl Glycol Copolymer
   Hersteller: Akzo-Nobel
34) Emery^{®} 1780
   INCI: Lanolin Alcohol
   Hersteller: Cognis Corporation (Emery)
35) Emulgade^{®}PL 68/50
   INCI: Cetearyl Glucoside, Cetearyl Alcohol
   Hersteller: Cognis Deutschland GmbH
36) Eumulgin^{®} B 2
   INCI: Ceteareth- 20
   Hersteller: Cognis Deutschland GmbH
37) Eumulgin^{®} VL 75
   INCI: Lauryl Glucoside (and) Polyglyceryl-2 Dipolyhydroxystearate (and) Glycerin
   Hersteller: Cognis Deutschland GmbH
38) Eusolex^{®} OCR
   INCI: Octocrylene
   Hersteller: Merck
39) Eusolex^{®} T 2000
   INCI: Titanium Dioxide, Alumina, Simethicone
   Hersteller: Rona (Merck)
40) Eutanol^{®} G
   INCI: Octyldodecanol
   Hersteller: Cognis Deutschland GmbH
41) Eutanol^{®}G 16
   INCI: Hexyldecanol
   Hersteller: Cognis Deutschland GmbH
42) Eutanol^{®}G 16 S
   INCI: Hexyldecyl Stearate
   Hersteller: Cognis Deutschland GmbH
43) Finsolv^{®} TN
   INCI: C 12/15 Alkyl Benzoate
   Hersteller: Findex (Nordmann/Rassmann)
44) Generol^{®} R
   INCI: Brassica Campestris (Rapseed) Sterols
   Hersteller: Cognis Deutschland GmbH
45) Glucate^{®} DO
   INCI: Methyl Glucose Dioleate
   Hersteller: NRC Nordmann/Rassmann
46) Hostaphat^{®} KL 340 N
   INCI: Trilaureth-4 Phosphate
   Hersteller: Clariant
47) lsolan^{®} PDI
   INCI: Diisostearoyl Polyglyceryl-3 Diisostearate
   Hersteller: Goldschmidt AG
48) Keltrol^{®} T
   INCI: Xanthan Gum
   Hersteller: CP Kelco
49) Lameform^{®} TGI
   INCI: Polyglyceryl-3 Diisostearate
   Hersteller: Cognis Deutschland GmbH
50) Lanette^{®} 14
   INCI: Myristyl Alcohol
   Hersteller: Cognis Deutschland GmbH
51) Lanette^{®} E
   INCI: Sodium Cetearyl Sulfate
   Hersteller: Cognis Deutschland GmbH
52) Lanette^{®}O
   INCI: Cetearyl Alcohol
   Hersteller: Cognis Deutschland GmbH
53) Monomuls^{®} 90-0-18
   INCI: Glyceryl Oleate
   Hersteller: Cognis Deutschland GmbH
54) Myrj^{®} 51
   INCI: PEG-30-Sterate
   Hersteller: Uniqema
55) Myritol^{®} 331
   INCI: Cocoglycerides
   Hersteller: Cognis Deutschland GmbH
56) Myritol^{®} PC
   INCI: Propylene Glycol Dicaprylate/Dicaprate
   Hersteller: Cognis Deutschland GmbH
57) Neo Heliopan^{®} 303
   INCI: Octocrylene
   Hersteller: Haarmann & Reimer
58) Neo Heliopan^{®} AV
   INCI: Ethylhexyl Methoxycinnamate
   Hersteller: Haarmann & Reimer
59) Neo Heliopan^{®} BB
   INCI: Benzophenone-3
   Hersteller: Haarmann & Reimer
60) Neo Heliopan^{®} E 1000
   INCI: Isoamyl-p-Methoxycinnamate
   Hersteller: Haarmann & Reimer
61) Neo Heliopan^{®} Hydro (Na-Salz)
   INCI: Phenylbenzimidazole Sulfonic Acid
   Hersteller: Haarmann & Reimer
62) Neo Heliopan^{®} MBC
   INCI: 4-Methylbenzylidene Camphor
   Hersteller: Haarmann & Reimer
63) Neo Heliopan^{®} OS
   INCI: Ethylhexyl Salicylate
   Hersteller: Haarmann & Reimer
64) Novata^{®} AB
   INCI: Cocoglycerides
   Hersteller: Cognis Deutschland GmbH
65) Parsol^{®} 1789
   INCI: Butyl Methoxydibenzoylmethane
   Hersteller: Hoffmann-La Roche (Givaudan)
66) Pemulen^{®} TR-2
   INCI: Acrylates / C10-30 Alkylacrylate Crosspolymer
   Hersteller: Goodrich
67) Photonyl^{®} LS
   INCI: Arginine, Disodium Adenosine Triphosphate, Mannitol, Pyridoxine HCL, Phenylalanine, Tyrosine
   Hersteller: Laboratoires Serobiologiques (Cognis)
68) Pripol^{®} 2033
   Dimerdiol
   Hersteller: Uniqema
69) Prisorine^{®} ISAC 3505
   INCI: Isostearic Acid
   Hersteller: Uniqema
70) Prisorine^{®} 3758
   INCI: Hydrogenated Polyisobutene
   Hersteller: Uniqema
73) SFE^{®} 839
   INCI: Cyclopentasiloxane and DimethiconeNinyl Dimethicone Crosspolymer
   Hersteller: GE Silicones
74) Silikonöl Wacker AK^{®} 350
   INCI: Dimethicone
   Hersteller: Wacker
75) Sovermol^{®} 908
   INCI: Dimerdiol
   Hersteller: Cognis Deutschland GmbH
76) Squatol^{®} S
   INCI: Hydrogenated Polyisobutene
   Hersteller: LCW (7-9 rue de l'Industrie 95310 St-Ouen l'Aumone France)
77) Tego^{®} Care 450
   INCI: Polyglyceryl-3 Methylglucose Distearate
   Hersteller: Tego Cosmetics (Goldschmidt)
78) Tego^{®} Care CG 90
   INCI: Cetearyl Glucoside
   Hersteller: Goldschmidt
79) Tween^{®} 60
   INCI: Polysorbate 60
   Hersteller: Uniqema (ICI Surfactants)
80) Uvinul^{®} T 150
   INCI: Octyl Triazone
   Hersteller: BASF
81) Veegum^{®} Ultra
   INCI: Magnesium Aluminium Silicate
   Hersteller: Vanderbilt
82) Z-Cote^{®} HP 1
   INCI: Zinc Oxide, Dimethicone
   Hersteller: BASF

## Patentansprüche

1. Verwendung von Dimerdiolen zur Verbesserung der Wasserfestigkeit von Sonnenschutzmittel, Zubereitungen der dekorativen Kosmetik, Insektenschutzmittel oder Deo/Antitranspirant-Formulierungen.

2. Verwendung gemäß Anspruch 1, **dadurch gekennzeichnet, dass** wenigstens ein Dimerdiol mit einer OH-Zahl von 200 - 220 verwendet wird.

3. Verwendung gemäß einem der Ansprüche 1 bis 2, **dadurch gekennzeichnet, dass** wenigstens ein Dimerdiol mit Jodzahlen von 20 bis 80, vorzugsweise 50 bis 70, verwendet wird.

## Claims

1. The use of dimer diols for improving the water resistance of sun protection preparations, decorative cosmetic products, insect repellents or deodorant/antiperspirant formulations.

2. Use according to claim 1, **characterized in that** at least one dimer diol with an OH value of 200 to 200 is used.

3. Use according to claim 1, **characterized in that** at least one dimer diol with iodine values of 20 to 80 and preferably 50 to 70 is used.

## Revendications

1. Utilisation de diols dimères pour améliorer l'inertie à l'eau d'agents de protection antisolaire, de préparations cosmétiques de décoration, d'agents de protection anti-insectes ou de formulations déodorantes/ antitranspirantes.

2. Utilisation selon la revendication 1,
**caractérisée en ce qu'**
on utilise au moins un diol dimère présentant un indice hydroxyle de 200-220.

3. Utilisation selon l'une des revendications 1 et 2,
**caractérisée en ce qu'**
on utilise au moins un diol dimère présentant des indices d'iode de 20 à 80, de préférence de 50 à 70.
